(19) Europäisches Patentamt
European Patent Office
Office européen des brevets

(11) **EP 3 512 562 B1**

(12) **EUROPEAN PATENT SPECIFICATION**

(45) Date of publication and mention
of the grant of the patent:
**30.06.2021   Bulletin 2021/26**

(51) Int Cl.:
**A61K 47/64** *(2017.01)*     **A61K 47/68** *(2017.01)*

(21) Application number: **17777504.6**

(86) International application number:
**PCT/EP2017/073245**

(22) Date of filing: **15.09.2017**

(87) International publication number:
**WO 2018/050808 (22.03.2018 Gazette 2018/12)**

(54) **ANTINEOPLASTIC FUSION PROTEIN**

ANTINEOPLASTISCHE FUSIONSPROTEINE

PROTÉINES DE FUSION ANTINÉOPLASIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **15.09.2016   PL 41871316**

(43) Date of publication of application:
**24.07.2019   Bulletin 2019/30**

(73) Proprietor: **Adamed Pharma S.A.**
**05-152 Czosnow (PL)**

(72) Inventor: **PAWLAK, Sebastian Dominik**
**01-100 Warszawa (PL)**

(74) Representative: **Matusiewicz, Katarzyna Joanna**
**Adamed Pharma S.A.**
**ul. Mariana Adamkiewicza 6A**
**Pienkow**
**05-152 Czosnów (PL)**

(56) References cited:
**WO-A1-2013/178783     WO-A1-2017/041143**
**WO-A1-2017/193059     WO-A2-2013/080147**
**US-A1- 2010 303 811**

SEQUENCE LISTING

<110> Adamed sp. z o.o. Pawlak, Sebastian Dominik

<120> Przeciwnowotworowe bia³ko fuzyjne

<130> Biol/KM/ONCOSTATM

<160> 13

<170> PatentIn version 3.5

<210> 1
<211> 551
<212> PRT
<213> Artificial Sequence

<220>
<223> fusion protein

<400> 1

```
Ala Ala Ile Gly Ser Cys Ser Lys Glu Tyr Arg Val Leu Leu Gly Gln
1               5                   10                  15


Leu Gln Lys Gln Thr Asp Leu Met Gln Asp Thr Ser Arg Leu Leu Asp
            20                  25                  30


Pro Tyr Ile Arg Ile Gln Gly Leu Asp Val Pro Lys Leu Arg Glu His
        35                  40                  45


Cys Arg Glu Arg Pro Gly Ala Phe Pro Ser Glu Glu Thr Leu Arg Gly
    50                  55                  60


Leu Gly Arg Arg Gly Phe Leu Gln Thr Leu Asn Ala Thr Leu Gly Cys
65                  70                  75                  80


Val Leu His Arg Leu Ala Asp Leu Glu Gln Arg Leu Pro Lys Ala Gln
                85                  90                  95


Asp Leu Glu Arg Ser Gly Leu Asn Ile Glu Asp Leu Glu Lys Leu Gln
            100                 105                 110


Met Ala Arg Pro Asn Ile Leu Gly Leu Arg Asn Asn Ile Tyr Cys Met
            115                 120                 125


Ala Gln Leu Leu Asp Asn Ser Asp Thr Ala Glu Pro Thr Lys Ala Gly
        130                 135                 140


Arg Gly Ala Ser Gln Pro Pro Thr Pro Thr Pro Ala Ser Asp Ala Phe
145                 150                 155                 160
```

Gln Arg Lys Leu Glu Gly Cys Arg Phe Leu His Gly Tyr His Arg Phe
                165             170             175

Met His Ser Val Gly Arg Val Phe Ser Lys Trp Gly Glu Ser Pro Asn
                180             185             190

Arg Ser Arg Arg Gly Gly Gly Gly Ser Ala Ser Gly Gly Pro Glu Gly
                195             200             205

Gly Ser Leu Ala Ala Leu Thr Ala His Gln Ala Cys His Leu Pro Leu
210             215             220

Glu Thr Phe Thr Arg His Arg Gln Pro Arg Gly Trp Glu Gln Leu Glu
225             230             235             240

Gln Cys Gly Tyr Pro Val Gln Arg Leu Val Ala Leu Tyr Leu Ala Ala
                245             250             255

Arg Leu Ser Trp Asn Gln Val Asp Gln Val Ile Ala Asn Ala Leu Ala
                260             265             270

Ser Pro Gly Ser Gly Gly Asp Leu Gly Glu Ala Ile Arg Glu Ser Pro
                275             280             285

Glu Gln Ala Arg Leu Ala Leu Thr Leu Ala Ala Ala Glu Ser Glu Arg
                290             295             300

Phe Val Arg Gln Gly Thr Gly Asn Asp Glu Ala Gly Ala Ala Asn Gly
305             310             315             320

Pro Ala Asp Ser Gly Asp Ala Leu Leu Glu Arg Asn Tyr Pro Thr Gly
                325             330             335

Ala Glu Phe Leu Gly Asp Gly Gly Asp Val Ser Phe Ser Thr Arg Gly
                340             345             350

Thr Gln Asn Trp Thr Val Glu Arg Leu Leu Gln Ala His Arg Gln Leu
                355             360             365

Glu Glu Ala Gly Tyr Val Phe Val Gly Tyr His Gly Thr Phe Leu Glu
                370             375             380

Ala Ala Gln Ser Ile Val Phe Gly Gly Val Arg Ala Arg Ser Gln Asp
385             390             395             400

Leu Asp Ala Ile Trp Ala Gly Phe Tyr Ile Ala Gly Asp Pro Ala Leu
                405             410             415

16

```
        Ala Tyr Gly Tyr Ala Gln Asp Gln Glu Pro Asp Ala Ala Gly Arg Ile
                    420                 425             430


        Arg Asn Gly Ala Leu Leu Arg Val Tyr Val Pro Arg Ser Ser Leu Pro
                    435                 440             445


        Gly Phe Tyr Ala Thr Ser Leu Thr Leu Ala Ala Pro Glu Ala Ala Gly
                    450                 455             460


        Glu Val Glu Arg Leu Ile Gly His Pro Leu Pro Leu Arg Leu Asp Ala
        465                 470                 475                 480


        Ile Thr Gly Pro Glu Glu Ser Gly Gly Arg Leu Glu Thr Ile Leu Gly
                        485                 490                 495


        Trp Pro Leu Ala Glu Arg Thr Val Val Ile Pro Ser Ala Ile Pro Thr
                    500                 505             510


        Asp Pro Arg Asn Val Gly Gly Asp Leu Asp Pro Ser Ser Ile Pro Asp
                    515                 520             525


        Ser Glu Gln Ala Ile Ser Ala Leu Pro Asp Tyr Ala Ser Gln Pro Gly
                530                 535                 540


        Lys Pro Pro Lys Asp Glu Leu
        545                 550
```

<210> 2
<211> 551
<212> PRT
<213> Artificial Sequence

<220>
<223> bia³ko fuzyjne

<400> 2

```
        Ala Ala Ile Gly Ser Cys Ser Lys Glu Tyr Arg Val Leu Leu Gly Gln
        1               5                   10                  15


        Leu Gln Lys Ala Thr Asp Leu Met Gln Asp Thr Ser Arg Leu Leu Asp
                    20                  25                  30


        Pro Tyr Ile Arg Ile Gln Gly Leu Asp Val Pro Lys Leu Arg Glu His
                    35                  40                  45


        Cys Arg Glu Arg Pro Gly Ala Phe Pro Ser Glu Glu Thr Leu Arg Gly
                50                  55                  60
```

Leu Gly Arg Arg Gly Phe Leu Gln Thr Leu Asn Ala Thr Leu Gly Cys
65              70              75              80

Val Leu His Arg Leu Ala Asp Leu Glu Gln Arg Leu Pro Lys Ala Gln
                85              90              95

Asp Leu Glu Arg Ser Gly Leu Asn Ile Glu Asp Leu Glu Lys Leu Gln
            100             105             110

Met Ala Arg Pro Asn Ile Leu Gly Leu Arg Asn Asn Ile Tyr Cys Met
            115             120             125

Ala Gln Leu Leu Asp Asn Ser Asp Thr Ala Glu Pro Thr Lys Ala Gly
        130             135             140

Arg Gly Ala Ser Gln Pro Pro Thr Pro Thr Pro Ala Ser Asp Ala Phe
145             150             155             160

Gln Arg Lys Leu Glu Gly Cys Arg Phe Leu His Gly Tyr His Arg Phe
            165             170             175

Met His Ser Val Gly Arg Val Phe Ser Lys Trp Gly Glu Ser Pro Asn
        180             185             190

Arg Ser Arg Arg Gly Gly Gly Gly Ser Ala Ser Gly Gly Pro Glu Gly
        195             200             205

Gly Ser Leu Ala Ala Leu Thr Ala His Gln Ala Cys His Leu Pro Leu
    210             215             220

Glu Thr Phe Thr Arg His Arg Gln Pro Arg Gly Trp Glu Gln Leu Glu
225             230             235             240

Gln Cys Gly Tyr Pro Val Gln Arg Leu Val Ala Leu Tyr Leu Ala Ala
            245             250             255

Arg Leu Ser Trp Asn Gln Val Asp Gln Val Ile Ala Asn Ala Leu Ala
        260             265             270

Ser Pro Gly Ser Gly Gly Asp Leu Gly Glu Ala Ile Arg Glu Ser Pro
    275             280             285

Glu Gln Ala Arg Leu Ala Leu Thr Leu Ala Ala Ala Glu Ser Glu Arg
    290             295             300

Phe Val Arg Gln Gly Thr Gly Asn Asp Glu Ala Gly Ala Ala Asn Gly
305             310             315             320

```
Pro Ala Asp Ser Gly Asp Ala Leu Leu Glu Arg Asn Tyr Pro Thr Gly
            325                 330             335

Ala Glu Phe Leu Gly Asp Gly Gly Asp Val Ser Phe Ser Thr Arg Gly
            340                 345             350

Thr Gln Asn Trp Thr Val Glu Arg Leu Leu Gln Ala His Arg Gln Leu
            355                 360             365

Glu Glu Ala Gly Tyr Val Phe Val Gly Tyr His Gly Thr Phe Leu Glu
    370             375             380

Ala Ala Gln Ser Ile Val Phe Gly Gly Val Arg Ala Arg Ser Gln Asp
385             390             395                 400

Leu Asp Ala Ile Trp Ala Gly Phe Tyr Ile Ala Gly Asp Pro Ala Leu
            405             410             415

Ala Tyr Gly Tyr Ala Gln Asp Gln Glu Pro Asp Ala Ala Gly Arg Ile
            420             425             430

Arg Asn Gly Ala Leu Leu Arg Val Tyr Val Pro Arg Ser Ser Leu Pro
            435             440             445

Gly Phe Tyr Ala Thr Ser Leu Thr Leu Ala Ala Pro Glu Ala Ala Gly
    450             455             460

Glu Val Glu Arg Leu Ile Gly His Pro Leu Pro Leu Arg Leu Asp Ala
465             470             475                 480

Ile Thr Gly Pro Glu Glu Ser Gly Gly Arg Leu Glu Thr Ile Leu Gly
            485             490             495

Trp Pro Leu Ala Glu Arg Thr Val Val Ile Pro Ser Ala Ile Pro Thr
            500             505             510

Asp Pro Arg Asn Val Gly Gly Asp Leu Asp Pro Ser Ser Ile Pro Asp
            515             520             525

Ser Glu Gln Ala Ile Ser Ala Leu Pro Asp Tyr Ala Ser Gln Pro Gly
    530             535             540

Lys Pro Pro Lys Asp Glu Leu
545             550
```

<210> 3
<211> 551

<212> PRT
<213> Artificial Sequence

<220>
<223> bia$^3$ko fuzyjne

<400> 3

<212> PRT
<213> Artificial Sequence

Ala Ala Ile Gly Ser Cys Ser Lys Glu Tyr Arg Val Leu Leu Gly Gln
1               5                   10                  15

Leu Gln Lys Gln Thr Asp Leu Met Gln Asp Thr Ser Arg Leu Leu Asp
            20                  25                  30

Pro Tyr Ile Arg Ile Gln Gly Leu Asp Val Pro Lys Leu Arg Glu His
            35                  40                  45

Cys Arg Glu Arg Pro Gly Ala Phe Pro Ser Glu Glu Thr Leu Arg Gly
    50                  55                  60

Leu Gly Arg Arg Gly Phe Leu Gln Thr Leu Asn Ala Thr Leu Gly Cys
65                  70                  75                  80

Val Leu His Arg Leu Ala Asp Leu Glu Gln Arg Leu Pro Lys Ala Gln
            85                  90                  95

Asp Leu Glu Arg Ser Gly Leu Asn Ile Glu Asp Leu Glu Lys Leu Gln
            100                 105                 110

Met Ala Arg Pro Asn Ile Leu Gly Leu Arg Asn Asn Ile Tyr Cys Met
            115                 120                 125

Ala Gln Leu Leu Asp Asn Ser Asp Thr Ala Glu Pro Thr Lys Ala Gly
    130                 135                 140

Arg Gly Ala Ser Gln Pro Pro Thr Pro Thr Pro Ala Ser Asp Ala Phe
145                 150                 155                 160

Gln Arg Ala Leu Glu Gly Cys Arg Phe Leu His Gly Tyr His Arg Phe
            165                 170                 175

Met His Ser Val Gly Arg Val Phe Ser Lys Trp Gly Glu Ser Pro Asn
            180                 185                 190

Arg Ser Arg Arg Gly Gly Gly Gly Ser Ala Ser Gly Gly Pro Glu Gly
            195                 200                 205

Gly Ser Leu Ala Ala Leu Thr Ala His Gln Ala Cys His Leu Pro Leu
    210                 215                 220

Glu Thr Phe Thr Arg His Arg Gln Pro Arg Gly Trp Glu Gln Leu Glu
225                 230             235                 240

Gln Cys Gly Tyr Pro Val Gln Arg Leu Val Ala Leu Tyr Leu Ala Ala
                245             250             255

Arg Leu Ser Trp Asn Gln Val Asp Gln Val Ile Ala Asn Ala Leu Ala
            260             265             270

Ser Pro Gly Ser Gly Gly Asp Leu Gly Glu Ala Ile Arg Glu Ser Pro
        275             280             285

Glu Gln Ala Arg Leu Ala Leu Thr Leu Ala Ala Ala Glu Ser Glu Arg
    290             295             300

Phe Val Arg Gln Gly Thr Gly Asn Asp Glu Ala Gly Ala Ala Asn Gly
305             310             315             320

Pro Ala Asp Ser Gly Asp Ala Leu Leu Glu Arg Asn Tyr Pro Thr Gly
            325             330             335

Ala Glu Phe Leu Gly Asp Gly Gly Asp Val Ser Phe Ser Thr Arg Gly
            340             345             350

Thr Gln Asn Trp Thr Val Glu Arg Leu Leu Gln Ala His Arg Gln Leu
        355             360             365

Glu Glu Ala Gly Tyr Val Phe Val Gly Tyr His Gly Thr Phe Leu Glu
    370             375             380

Ala Ala Gln Ser Ile Val Phe Gly Gly Val Arg Ala Arg Ser Gln Asp
385             390             395             400

Leu Asp Ala Ile Trp Ala Gly Phe Tyr Ile Ala Gly Asp Pro Ala Leu
            405             410             415

Ala Tyr Gly Tyr Ala Gln Asp Gln Glu Pro Asp Ala Ala Gly Arg Ile
        420             425             430

Arg Asn Gly Ala Leu Leu Arg Val Tyr Val Pro Arg Ser Ser Leu Pro
        435             440             445

Gly Phe Tyr Ala Thr Ser Leu Thr Leu Ala Ala Pro Glu Ala Ala Gly
        450             455             460

Glu Val Glu Arg Leu Ile Gly His Pro Leu Pro Leu Arg Leu Asp Ala
465             470             475             480

```
        Ile Thr Gly Pro Glu Glu Ser Gly Gly Arg Leu Glu Thr Ile Leu Gly
                        485             490             495

        Trp Pro Leu Ala Glu Arg Thr Val Val Ile Pro Ser Ala Ile Pro Thr
                        500             505             510

        Asp Pro Arg Asn Val Gly Gly Asp Leu Asp Pro Ser Ser Ile Pro Asp
                        515             520             525

        Ser Glu Gln Ala Ile Ser Ala Leu Pro Asp Tyr Ala Ser Gln Pro Gly
                        530             535             540

        Lys Pro Pro Lys Asp Glu Leu
        545             550
```

<210> 4
<211> 551
<212> PRT
<213> Artificial Sequence

<220>
<223> bia³ko fuzyjne

<400> 4

```
        Ala Ala Ile Gly Ser Cys Ser Lys Glu Tyr Arg Val Leu Leu Gly Gln
        1               5               10              15

        Leu Gln Lys Ala Thr Asp Leu Met Gln Asp Thr Ser Arg Leu Leu Asp
                        20              25              30

        Pro Tyr Ile Arg Ile Gln Gly Leu Asp Val Pro Lys Leu Arg Glu His
                        35              40              45

        Cys Arg Glu Arg Pro Gly Ala Phe Pro Ser Glu Glu Thr Leu Arg Gly
                50              55              60

        Leu Gly Arg Arg Gly Phe Leu Gln Thr Leu Asn Ala Thr Leu Gly Cys
        65                      70              75              80

        Val Leu His Arg Leu Ala Asp Leu Glu Gln Arg Leu Pro Lys Ala Gln
                        85              90              95

        Asp Leu Glu Arg Ser Gly Leu Asn Ile Glu Asp Leu Glu Lys Leu Gln
                        100             105             110

        Met Ala Arg Pro Asn Ile Leu Gly Leu Arg Asn Asn Ile Tyr Cys Met
                        115             120             125
```

```
Ala Gln Leu Leu Asp Asn Ser Asp Thr Ala Glu Pro Thr Lys Ala Gly
    130                 135                 140

Arg Gly Ala Ser Gln Pro Pro Thr Pro Thr Pro Ala Ser Asp Ala Phe
145                 150                 155                 160

Gln Arg Ala Leu Glu Gly Cys Arg Phe Leu His Gly Tyr His Arg Phe
                165                 170                 175

Met His Ser Val Gly Arg Val Phe Ser Lys Trp Gly Glu Ser Pro Asn
            180                 185                 190

Arg Ser Arg Arg Gly Gly Gly Gly Ser Ala Ser Gly Gly Pro Glu Gly
        195                 200                 205

Gly Ser Leu Ala Ala Leu Thr Ala His Gln Ala Cys His Leu Pro Leu
    210                 215                 220

Glu Thr Phe Thr Arg His Arg Gln Pro Arg Gly Trp Glu Gln Leu Glu
225                 230                 235                 240

Gln Cys Gly Tyr Pro Val Gln Arg Leu Val Ala Leu Tyr Leu Ala Ala
                245                 250                 255

Arg Leu Ser Trp Asn Gln Val Asp Gln Val Ile Ala Asn Ala Leu Ala
            260                 265                 270

Ser Pro Gly Ser Gly Gly Asp Leu Gly Glu Ala Ile Arg Glu Ser Pro
        275                 280                 285

Glu Gln Ala Arg Leu Ala Leu Thr Leu Ala Ala Ala Glu Ser Glu Arg
    290                 295                 300

Phe Val Arg Gln Gly Thr Gly Asn Asp Glu Ala Gly Ala Ala Asn Gly
305                 310                 315                 320

Pro Ala Asp Ser Gly Asp Ala Leu Leu Glu Arg Asn Tyr Pro Thr Gly
                325                 330                 335

Ala Glu Phe Leu Gly Asp Gly Gly Asp Val Ser Phe Ser Thr Arg Gly
            340                 345                 350

Thr Gln Asn Trp Thr Val Glu Arg Leu Leu Gln Ala His Arg Gln Leu
        355                 360                 365

Glu Glu Ala Gly Tyr Val Phe Val Gly Tyr His Gly Thr Phe Leu Glu
    370                 375                 380
```

24

```
Ala Ala Gln Ser Ile Val Phe Gly Gly Val Arg Ala Arg Ser Gln Asp
385             390             395             400

Leu Asp Ala Ile Trp Ala Gly Phe Tyr Ile Ala Gly Asp Pro Ala Leu
            405             410             415

Ala Tyr Gly Tyr Ala Gln Asp Gln Glu Pro Asp Ala Ala Gly Arg Ile
        420             425             430

Arg Asn Gly Ala Leu Leu Arg Val Tyr Val Pro Arg Ser Ser Leu Pro
        435             440             445

Gly Phe Tyr Ala Thr Ser Leu Thr Leu Ala Ala Pro Glu Ala Ala Gly
    450             455             460

Glu Val Glu Arg Leu Ile Gly His Pro Leu Pro Leu Arg Leu Asp Ala
465             470             475             480

Ile Thr Gly Pro Glu Glu Ser Gly Gly Arg Leu Glu Thr Ile Leu Gly
            485             490             495

Trp Pro Leu Ala Glu Arg Thr Val Val Ile Pro Ser Ala Ile Pro Thr
        500             505             510

Asp Pro Arg Asn Val Gly Gly Asp Leu Asp Pro Ser Ser Ile Pro Asp
        515             520             525

Ser Glu Gln Ala Ile Ser Ala Leu Pro Asp Tyr Ala Ser Gln Pro Gly
    530             535             540

Lys Pro Pro Lys Asp Glu Leu
545             550
```

<210> 5
<211> 543
<212> PRT
<213> Artificial Sequence

<220>
<223> bia³ko fuzyjne

<400> 5

```
Ala Ala Ile Gly Ser Cys Ser Lys Glu Tyr Arg Val Leu Leu Gly Gln
1             5             10              15

Leu Gln Lys Gln Thr Asp Leu Met Gln Asp Thr Ser Arg Leu Leu Asp
        20              25              30
```

25

```
Pro Tyr Ile Arg Ile Gln Gly Leu Asp Val Pro Lys Leu Arg Glu His
        35                  40              45

Cys Arg Glu Arg Pro Gly Ala Phe Pro Ser Glu Glu Thr Leu Arg Gly
        50                  55              60

Leu Gly Arg Arg Gly Phe Leu Gln Thr Leu Asn Ala Thr Leu Gly Cys
65                  70                  75                  80

Val Leu His Arg Leu Ala Asp Leu Glu Gln Arg Leu Gly Gly Gly Asn
                85                  90                  95

Ile Glu Asp Leu Glu Lys Leu Gln Met Ala Arg Pro Asn Ile Leu Gly
                100                 105                 110

Leu Arg Asn Asn Ile Tyr Cys Met Ala Gln Leu Leu Asp Asn Ser Asp
                115                 120                 125

Thr Ala Glu Pro Thr Lys Ala Gly Arg Gly Ala Ser Gln Pro Pro Thr
        130                 135                 140

Pro Thr Pro Ala Ser Asp Ala Phe Gln Arg Lys Leu Glu Gly Cys Arg
145                 150                 155                 160

Phe Leu His Gly Tyr His Arg Phe Met His Ser Val Gly Arg Val Phe
                165                 170                 175

Ser Lys Trp Gly Glu Ser Pro Asn Arg Ser Arg Arg Gly Gly Gly Gly
                180                 185                 190

Ser Ala Ser Gly Gly Pro Glu Gly Gly Ser Leu Ala Ala Leu Thr Ala
        195                 200                 205

His Gln Ala Cys His Leu Pro Leu Glu Thr Phe Thr Arg His Arg Gln
        210                 215                 220

Pro Arg Gly Trp Glu Gln Leu Glu Gln Cys Gly Tyr Pro Val Gln Arg
225                 230                 235                 240

Leu Val Ala Leu Tyr Leu Ala Ala Arg Leu Ser Trp Asn Gln Val Asp
                245                 250                 255

Gln Val Ile Ala Asn Ala Leu Ala Ser Pro Gly Ser Gly Gly Asp Leu
                260                 265                 270

Gly Glu Ala Ile Arg Glu Ser Pro Glu Gln Ala Arg Leu Ala Leu Thr
                275                 280                 285
```

```
Leu Ala Ala Ala Glu Ser Glu Arg Phe Val Arg Gln Gly Thr Gly Asn
    290             295             300

Asp Glu Ala Gly Ala Ala Asn Gly Pro Ala Asp Ser Gly Asp Ala Leu
305             310             315             320

Leu Glu Arg Asn Tyr Pro Thr Gly Ala Glu Phe Leu Gly Asp Gly Gly
            325             330             335

Asp Val Ser Phe Ser Thr Arg Gly Thr Gln Asn Trp Thr Val Glu Arg
            340             345             350

Leu Leu Gln Ala His Arg Gln Leu Glu Glu Ala Gly Tyr Val Phe Val
            355             360             365

Gly Tyr His Gly Thr Phe Leu Glu Ala Ala Gln Ser Ile Val Phe Gly
    370             375             380

Gly Val Arg Ala Arg Ser Gln Asp Leu Asp Ala Ile Trp Ala Gly Phe
385             390             395             400

Tyr Ile Ala Gly Asp Pro Ala Leu Ala Tyr Gly Tyr Ala Gln Asp Gln
            405             410             415

Glu Pro Asp Ala Ala Gly Arg Ile Arg Asn Gly Ala Leu Leu Arg Val
            420             425             430

Tyr Val Pro Arg Ser Ser Leu Pro Gly Phe Tyr Ala Thr Ser Leu Thr
            435             440             445

Leu Ala Ala Pro Glu Ala Ala Gly Glu Val Glu Arg Leu Ile Gly His
    450             455             460

Pro Leu Pro Leu Arg Leu Asp Ala Ile Thr Gly Pro Glu Glu Ser Gly
465             470             475             480

Gly Arg Leu Glu Thr Ile Leu Gly Trp Pro Leu Ala Glu Arg Thr Val
            485             490             495

Val Ile Pro Ser Ala Ile Pro Thr Asp Pro Arg Asn Val Gly Gly Asp
            500             505             510

Leu Asp Pro Ser Ser Ile Pro Asp Ser Glu Gln Ala Ile Ser Ala Leu
            515             520             525

Pro Asp Tyr Ala Ser Gln Pro Gly Lys Pro Pro Lys Asp Glu Leu
```

530                    535                    540

<210> 6
<211> 196
<212> PRT
<213> Homo sapiens

<400> 6

Ala Ala Ile Gly Ser Cys Ser Lys Glu Tyr Arg Val Leu Leu Gly Gln
1               5                   10              15

Leu Gln Lys Gln Thr Asp Leu Met Gln Asp Thr Ser Arg Leu Leu Asp
            20                  25                  30

Pro Tyr Ile Arg Ile Gln Gly Leu Asp Val Pro Lys Leu Arg Glu His
        35                  40                  45

Cys Arg Glu Arg Pro Gly Ala Phe Pro Ser Glu Glu Thr Leu Arg Gly
    50                  55                  60

Leu Gly Arg Arg Gly Phe Leu Gln Thr Leu Asn Ala Thr Leu Gly Cys
65                  70                  75                  80

Val Leu His Arg Leu Ala Asp Leu Glu Gln Arg Leu Pro Lys Ala Gln
                85                  90                  95

Asp Leu Glu Arg Ser Gly Leu Asn Ile Glu Asp Leu Glu Lys Leu Gln
            100                 105                 110

Met Ala Arg Pro Asn Ile Leu Gly Leu Arg Asn Asn Ile Tyr Cys Met
            115                 120                 125

Ala Gln Leu Leu Asp Asn Ser Asp Thr Ala Glu Pro Thr Lys Ala Gly
    130                 135                 140

Arg Gly Ala Ser Gln Pro Pro Thr Pro Thr Pro Ala Ser Asp Ala Phe
145                 150                 155                 160

Gln Arg Lys Leu Glu Gly Cys Arg Phe Leu His Gly Tyr His Arg Phe
                165                 170                 175

Met His Ser Val Gly Arg Val Phe Ser Lys Trp Gly Glu Ser Pro Asn
            180                 185                 190

Arg Ser Arg Arg
            195

<210> 7
<211> 196
<212> PRT
<213> Homo sapiens

<400> 7

```
Ala Ala Ile Gly Ser Cys Ser Lys Glu Tyr Arg Val Leu Leu Gly Gln
1               5                   10                  15

Leu Gln Lys Ala Thr Asp Leu Met Gln Asp Thr Ser Arg Leu Leu Asp
            20                  25                  30

Pro Tyr Ile Arg Ile Gln Gly Leu Asp Val Pro Lys Leu Arg Glu His
            35                  40                  45

Cys Arg Glu Arg Pro Gly Ala Phe Pro Ser Glu Glu Thr Leu Arg Gly
        50                  55                  60

Leu Gly Arg Arg Gly Phe Leu Gln Thr Leu Asn Ala Thr Leu Gly Cys
65                  70                  75                  80

Val Leu His Arg Leu Ala Asp Leu Glu Gln Arg Leu Pro Lys Ala Gln
                85                  90                  95

Asp Leu Glu Arg Ser Gly Leu Asn Ile Glu Asp Leu Glu Lys Leu Gln
            100                 105                 110

Met Ala Arg Pro Asn Ile Leu Gly Leu Arg Asn Asn Ile Tyr Cys Met
            115                 120                 125

Ala Gln Leu Leu Asp Asn Ser Asp Thr Ala Glu Pro Thr Lys Ala Gly
        130                 135                 140

Arg Gly Ala Ser Gln Pro Pro Thr Pro Thr Pro Ala Ser Asp Ala Phe
145                 150                 155                 160

Gln Arg Lys Leu Glu Gly Cys Arg Phe Leu His Gly Tyr His Arg Phe
                165                 170                 175

Met His Ser Val Gly Arg Val Phe Ser Lys Trp Gly Glu Ser Pro Asn
            180                 185                 190

Arg Ser Arg Arg
            195
```

<210> 8
<211> 196
<212> PRT

<213> Homo sapiens

<400> 8

|     |     |     |     |     |     |     |     |     |     |     |     |     |     |     |     |
|-----|-----|-----|-----|-----|-----|-----|-----|-----|-----|-----|-----|-----|-----|-----|-----|
| Ala | Ala | Ile | Gly | Ser | Cys | Ser | Lys | Glu | Tyr | Arg | Val | Leu | Leu | Gly | Gln |
| 1   |     |     |     | 5   |     |     |     |     | 10  |     |     |     |     | 15  |     |

Leu Gln Lys Gln Thr Asp Leu Met Gln Asp Thr Ser Arg Leu Leu Asp
              20              25                  30

Pro Tyr Ile Arg Ile Gln Gly Leu Asp Val Pro Lys Leu Arg Glu His
          35              40              45

Cys Arg Glu Arg Pro Gly Ala Phe Pro Ser Glu Glu Thr Leu Arg Gly
      50              55              60

Leu Gly Arg Arg Gly Phe Leu Gln Thr Leu Asn Ala Thr Leu Gly Cys
65              70              75              80

Val Leu His Arg Leu Ala Asp Leu Glu Gln Arg Leu Pro Lys Ala Gln
              85              90              95

Asp Leu Glu Arg Ser Gly Leu Asn Ile Glu Asp Leu Glu Lys Leu Gln
          100             105             110

Met Ala Arg Pro Asn Ile Leu Gly Leu Arg Asn Asn Ile Tyr Cys Met
          115             120             125

Ala Gln Leu Leu Asp Asn Ser Asp Thr Ala Glu Pro Thr Lys Ala Gly
      130             135             140

Arg Gly Ala Ser Gln Pro Pro Thr Pro Thr Pro Ala Ser Asp Ala Phe
145             150             155             160

Gln Arg Ala Leu Glu Gly Cys Arg Phe Leu His Gly Tyr His Arg Phe
              165             170             175

Met His Ser Val Gly Arg Val Phe Ser Lys Trp Gly Glu Ser Pro Asn
          180             185             190

Arg Ser Arg Arg
          195

<210> 9
<211> 196
<212> PRT
<213> Homo sapiens

<400> 9

```
Ala Ala Ile Gly Ser Cys Ser Lys Glu Tyr Arg Val Leu Leu Gly Gln
1               5               10              15

Leu Gln Lys Ala Thr Asp Leu Met Gln Asp Thr Ser Arg Leu Leu Asp
            20              25              30

Pro Tyr Ile Arg Ile Gln Gly Leu Asp Val Pro Lys Leu Arg Glu His
        35              40              45

Cys Arg Glu Arg Pro Gly Ala Phe Pro Ser Glu Glu Thr Leu Arg Gly
    50              55              60

Leu Gly Arg Arg Gly Phe Leu Gln Thr Leu Asn Ala Thr Leu Gly Cys
65              70              75              80

Val Leu His Arg Leu Ala Asp Leu Glu Gln Arg Leu Pro Lys Ala Gln
            85              90              95

Asp Leu Glu Arg Ser Gly Leu Asn Ile Glu Asp Leu Glu Lys Leu Gln
        100             105             110

Met Ala Arg Pro Asn Ile Leu Gly Leu Arg Asn Asn Ile Tyr Cys Met
        115             120             125

Ala Gln Leu Leu Asp Asn Ser Asp Thr Ala Glu Pro Thr Lys Ala Gly
        130             135             140

Arg Gly Ala Ser Gln Pro Pro Thr Pro Thr Pro Ala Ser Asp Ala Phe
145             150             155             160

Gln Arg Ala Leu Glu Gly Cys Arg Phe Leu His Gly Tyr His Arg Phe
            165             170             175

Met His Ser Val Gly Arg Val Phe Ser Lys Trp Gly Glu Ser Pro Asn
        180             185             190

Arg Ser Arg Arg
        195
```

<210> 10
<211> 188
<212> PRT
<213> Homo sapiens

<400> 10

```
Ala Ala Ile Gly Ser Cys Ser Lys Glu Tyr Arg Val Leu Leu Gly Gln
1               5                   10                  15

Leu Gln Lys Gln Thr Asp Leu Met Gln Asp Thr Ser Arg Leu Leu Asp
            20                  25                  30

Pro Tyr Ile Arg Ile Gln Gly Leu Asp Val Pro Lys Leu Arg Glu His
        35                  40                  45

Cys Arg Glu Arg Pro Gly Ala Phe Pro Ser Glu Glu Thr Leu Arg Gly
    50                  55                  60

Leu Gly Arg Arg Gly Phe Leu Gln Thr Leu Asn Ala Thr Leu Gly Cys
65                  70                  75                  80

Val Leu His Arg Leu Ala Asp Leu Glu Gln Arg Leu Gly Gly Gly Asn
                85                  90                  95

Ile Glu Asp Leu Glu Lys Leu Gln Met Ala Arg Pro Asn Ile Leu Gly
            100                 105                 110

Leu Arg Asn Asn Ile Tyr Cys Met Ala Gln Leu Leu Asp Asn Ser Asp
        115                 120                 125

Thr Ala Glu Pro Thr Lys Ala Gly Arg Gly Ala Ser Gln Pro Pro Thr
    130                 135                 140

Pro Thr Pro Ala Ser Asp Ala Phe Gln Arg Lys Leu Glu Gly Cys Arg
145                 150                 155                 160

Phe Leu His Gly Tyr His Arg Phe Met His Ser Val Gly Arg Val Phe
                165                 170                 175

Ser Lys Trp Gly Glu Ser Pro Asn Arg Ser Arg Arg
            180                 185
```

<210> 11
<211> 342
<212> PRT
<213> Pseudomonas aeruginosa

<400> 11

Pro Glu Gly Gly Ser Leu Ala Ala Leu Thr Ala His Gln Ala Cys His
1                   5                   10                  15

Leu Pro Leu Glu Thr Phe Thr Arg His Arg Gln Pro Arg Gly Trp Glu
              20                  25                  30

Gln Leu Glu Gln Cys Gly Tyr Pro Val Gln Arg Leu Val Ala Leu Tyr
              35                  40                  45

Leu Ala Ala Arg Leu Ser Trp Asn Gln Val Asp Gln Val Ile Ala Asn
              50                  55                  60

```
Ala Leu Ala Ser Pro Gly Ser Gly Gly Asp Leu Gly Glu Ala Ile Arg
65              70              75              80

Glu Ser Pro Glu Gln Ala Arg Leu Ala Leu Thr Leu Ala Ala Ala Glu
                85              90              95

Ser Glu Arg Phe Val Arg Gln Gly Thr Gly Asn Asp Glu Ala Gly Ala
            100             105             110

Ala Asn Gly Pro Ala Asp Ser Gly Asp Ala Leu Leu Glu Arg Asn Tyr
            115             120             125

Pro Thr Gly Ala Glu Phe Leu Gly Asp Gly Gly Asp Val Ser Phe Ser
        130             135             140

Thr Arg Gly Thr Gln Asn Trp Thr Val Glu Arg Leu Leu Gln Ala His
145             150             155             160

Arg Gln Leu Glu Glu Ala Gly Tyr Val Phe Val Gly Tyr His Gly Thr
            165             170             175

Phe Leu Glu Ala Ala Gln Ser Ile Val Phe Gly Gly Val Arg Ala Arg
            180             185             190

Ser Gln Asp Leu Asp Ala Ile Trp Ala Gly Phe Tyr Ile Ala Gly Asp
            195             200             205

Pro Ala Leu Ala Tyr Gly Tyr Ala Gln Asp Gln Glu Pro Asp Ala Ala
        210             215             220

Gly Arg Ile Arg Asn Gly Ala Leu Leu Arg Val Tyr Val Pro Arg Ser
225             230             235             240

Ser Leu Pro Gly Phe Tyr Ala Thr Ser Leu Thr Leu Ala Ala Pro Glu
            245             250             255

Ala Ala Gly Glu Val Glu Arg Leu Ile Gly His Pro Leu Pro Leu Arg
            260             265             270

Leu Asp Ala Ile Thr Gly Pro Glu Glu Ser Gly Gly Arg Leu Glu Thr
        275             280             285

Ile Leu Gly Trp Pro Leu Ala Glu Arg Thr Val Val Ile Pro Ser Ala
        290             295             300

Ile Pro Thr Asp Pro Arg Asn Val Gly Gly Asp Leu Asp Pro Ser Ser
```

305                310                315                320

```
Ile Pro Asp Ser Glu Gln Ala Ile Ser Ala Leu Pro Asp Tyr Ala Ser
                    325                 330                 335

Gln Pro Gly Lys Pro Pro
            340
```

<210> 12
<211> 9
<212> PRT
<213> Artificial Sequence

<220>
<223> linker steryczny

<400> 12

```
                    Gly Gly Gly Gly Ser Ala Ser Gly Gly
                    1               5
```

<210> 13
<211> 4
<212> PRT
<213> Artificial Sequence

<220>
<223> motyw kieruj$^1$cy do retikulum

<400> 13

```
Lys Asp Glu Leu
1
```

**Description**

The invention relates to the field of therapeutic recombinant fusion proteins. Specifically, the invention relates to fusion proteins of Pseudomonas exotoxin A in combination with a human oncostatin M (OSM) fragment or a mutant form of an oncostatin M fragment, to a mutant form of an oncostatin M fragment, and to the use thereof in antineoplastic therapy.

*Pseudomonas aeruginosa* exotoxin A is a polypeptide comprised of three functional domains, the first of which is responsible for the recognition and binding it to the cell surface, the second for the translocation from the endosome to the cytoplasm, and the third has toxic enzymatic activity. After binding to the cell, it is transported to the cell interior as a protoxin, and then, in the endosomal environment, it is proteolytically activated by the proteolytic enzyme furin which cleaves the polypeptide chain to form a 28 kDa N-terminal fragment and a toxic polypeptide 37 kDa. In the cytoplasm, it recognises the elongation factor 2 (EF-2), and inactivates it by carrying out the ADP-ribosylation thereof. The exhaustion of the cellular EF-2 leads to the inhibition of protein synthesis in the cell and to death thereof.

Cytotoxicity of Pseudomonas exotoxin A towards many types of neoplastic cells has been described in detail. There are also known approaches in which Pseudomonas exotoxin A was used as a fusion with other proteins. In order to overcome this systemic cytotoxicity, various targeted therapies aimed at directing Pseudomonas toxins to specific types of cells and tissues have been designed. The targeted transport of bacterial toxins, including the Pseudomonas exotoxin, is known and described, for example by W. Debinski et al. J Biol Chem. 1995 Jul 14;270(28):16775-80. A novel chimeric protein composed of IL13 and Pseudomonas exotoxin is highly toxic to human carcinoma cells expressing receptors to IL13 and IL4. Fusions of the Pseudomonas exotoxin with IL-13 are also undergoing clinical trials in metastatic diseases: Liu-Chittenden Y, Cancer Med. 2015 Jul;4(7):1060-8. doi: 10.1002/cam4.449. Epub 2015 Mar 13.Phase I trial of systemic intravenous infusion of interleukin-13-Pseudomonas exotoxin in patients with metastatic adrenocortical carcinoma.

The activity of the fusion of a mutant form of Pseudomonas toxin with interleukin 6 in relation to AML cells isolated from paediatric patients has also been studied. Boayue KB et al. Leukemia. 1998 Feb;12(2):182-91. Pediatric acute myelogenous leukemia cells express IL-6 receptors and are sensitive to a recombinant IL6-Pseudomonas exotoxin.

Moreover, a fusion protein consisting of interleukin 6 and a mutant form of Pseudomonas exotoxin (IL6-PE40), provided with the targeting domain KDEL has also been described. This fusion was described as cytotoxic towards the multiple myeloma cells (Cui JW et al., 2004 Dec;12(6):825-8. Biochemical and physical properties for a recombinant IL6 Pseudomonas exotoxin fusion protein IL6D24-PE40KDEL).

However, in the above approaches, the possibility has been demonstrated for directing the Pseudomonas toxin only to neoplastic cells expressing receptors for the indicated interleukins, while the cells devoid of such receptors cannot be a therapeutic target. Moreover, the interleukins used as carriers for Pseudomonas toxins on certain types of cells induce the proliferation, which results in an increase of neoplasms or in toxicity.

Therefore, the technical problem lies in the lack of an effective system of directing the Pseudomonas toxin to various types of neoplastic cells i.e. a system with a significantly broader binding specificity obtained through the interaction with more than one type of overexpressed receptors on neoplastic cells. Another technical problem lies in the lack of an effective system of directing the Pseudomonas toxin to neoplastic cells that do not overexpress the receptor for the toxin carrier, or to neoplastic cells towards which the toxin carrier has a stimulating activity or a toxic effect.

Therefore, the object of the present invention is to develop an effective, targeted transport of the Pseudomonas exotoxin A, which will result in broad specificity of binding with neoplastic cells, and consequently in directing the Pseudomonas toxin to various types of target neoplastic cells, wherein this targeted transport ensures either significant or complete limitation to the activity towards normal cells, and enables directing the Pseudomonas toxin to neoplastic cells that do not overexpress the receptor for the toxin carrier, or to neoplastic cells towards which the toxin carrier has a stimulating activity or a toxic effect.

This problem is solved by the present invention through the use of human cytokine Oncostatin M (OSM) and mutant forms of oncostatin M as neoplasm-specific carriers of molecules or domains with known cytotoxic activity, in particular the Pseudomonas exotoxin A.

In particular, this problem has been solved through the provision of fusion proteins according to the invention OSMPE1.0 of SEQ. No 1, OSMPE1.1 of SEQ. No 2, OSMPE1.2 of SEQ. No 3, OSMPE1.3 of SEQ. No 4, OSMPE2.0 of SEQ. No 5, which are described in detail, respectively, in Embodiments 1 to 5.

To date, oncostatin M itself has been considered as a potential antiproliferative agent in case of breast or lung neoplasms, neuromas, or certain melanomas (Zarling JM et al., Oncostatin M: a growth regulator produced by differentiated histiocytic lymphoma cells. Proc Natl Acad Sci U S A. 1986 Dec;83(24):9739-43, Horn et al., 1990). However, due to numerous other functions of this cytokine, associated with the proinflammatory activity or even stimulating carcinogenesis (David E et al., Oncostatin M is a growth factor for Ewing sarcoma. Am J Pathol. 2012 Nov; 181(5):1782-95), the use thereof did not augur a significant possibility of an advantage over other potential therapeutics, and even generated additional hazards associated with the safety of a therapy.

OSM belongs to the IL6 family, and targets the gp130 receptor overexpressed on neoplastic cells.

The activity of oncostatin is closely related to the presence of OSMR receptors (Lacreusette et al., 2007). The OSMR receptor is present on many types of normal cells (Tamura S. et al., Developmental expression pattern of oncostatin M receptor beta in mice. Mech Dev. 2002 Jul; 115(1-2):127-31); however, the overexpression thereof is only associated with pathological conditions. It has been demonstrated that the OSMR levels are significantly elevated on many types of neoplastic cells, *inter alia* of breasts and the prostate (Tanaka M et al., A multifunctional cytokine. Rev Physiol Biochem Pharmacol. 2003;149:39-52), or the uterine cervix (Caffarel MM et al., Oncostatin M receptor is a novel therapeutic target in cervical squamous cell carcinoma. J Pathol. 2014 Mar; 232(4):386-90).

OSM receptors share one site of interaction within the OSM structure with the LIFR receptors. Both these receptors serve regulatory functions.

Oncostatin M also binds the glycoprotein membrane receptor gp130. This is a common receptor for ligands from the IL-6 family, involved in the activation of many signaling pathways, *inter alia* MAPK, JAK/STAT3, PI3'K, which serve significant roles in the immunological response, inflammations, proliferation, cell differentiation, and carcinogenesis. Such an arrangement generates two types of complexes of oncostatin with receptors, namely gp130/LIFR or gp130/OSMR.

The mode of forming complexes with receptors is as follows: OSM individually binds the gp130 receptor, and such a complex has no biological activity until the third component i.e. the LIFR or OSMR receptors have been bound (Liu J. et al., Interactions between oncostatin M and the IL-6 signal transducer, gp130. Cytokine. 1994 May;6(3):272-8).

Due to the binding with several receptors, oncostatin M has broad specificity for binding with neoplastic cells. As a result of the ability to form active complexes with two types of receptors, OSM exhibits pleiotropic activity and multidirectionality of action.

Oncostatin M and the receptors thereof, due to their various functions, serve a significant role in the physiology of neoplastic cells. Initially, OSM was described as an agent inhibiting *in vitro* the growth of melanoma cells (Zarling JM et al., Oncostatin M: a growth regulator produced by differentiated histiocytic lymphoma cells. Proc Natl Acad Sci U S A. 1986 Dec;83(24):9739-43). The effective inhibition of proliferation by OSM on many types of neoplastic cells was demonstrated e.g. a panel of lung neoplastic cells, (Horn D. et al., Regulation of cell growth by recombinant oncostatin M. Growth Factors. 1990;2(2-3):157-65), breast (Li C et al., Induction of S100A9 gene expression by cytokine oncostatin M in breast cancer cells through the STAT3 signaling cascade. Breast Cancer Res Treat. 2004 Sep;87(2):123-34), neuromas (Zarling JM et al., Oncostatin M: a growth regulator produced by differentiated histiocytic lymphoma cells. Proc Natl Acad Sci U S A. 1986 Dec; 83(24):9739-43) and bones (David E et al., Direct anticancer effect of oncostatin M on chondrosarcoma. Int J Cancer. 2011 Apr 15;128(8):1822-35). However, OSM activity is not unidirectional, and for the types of neoplastic lines other than those indicated above, OSM often exhibited the opposite effect i.e. stimulation of proliferation. Such an effect was described for prostatic neoplasms (Godoy-Tundidor S. et al., Interleukin-6 and oncostatin M stimulation of proliferation of prostate cancer 22Rv1 cells through the signaling pathways of p38 mitogen-activated protein kinase and phosphatidylinositol 3-kinase. Prostate. 2005 Jul 1;64(2):209-16), ovary (Li Q et al., Oncostatin M promotes proliferation of ovarian cancer cells through signal transducer and activator of transcription 3. Int J Mol Med. 2011 Jul;28(1):101-8), and Ewing's sarcoma (David E et al., Oncostatin M is a growth factor for Ewing sarcoma. Am J Pathol. 2012 Nov;181(5):1782-95). Although OSM and the mutant forms thereof as such have not proved attractive enough as an antineoplastic agent, due to the affinity for more than one overexpressed effector they may serve as an effective carrier directing toxic molecules to neoplastic cells.

Oncostatin M may serve as a targeting carrier for any protein molecule exhibiting cytotoxic activity. In particular, it may serve as a carrier of the Pseudomonas toxin, in particular of the Pseudomonas toxin fragment that only includes the enzymatic part (domain III) and a fragment of the translocation domain. Such a fragment of the Pseudomonas toxin is not able to specifically bind to the cell, therefore the action thereof should only become apparent after the endocytosis mediated by oncostatin M bound on the cell with at least one specific receptor.

Detailed description of the invention

The essence of the invention is the use of human Oncostatin M (OSM) and mutant forms of oncostatin M as carriers and supporting effectors for cytotoxic domains in antineoplastic therapy. The invention consists in combining two protein domains: Human cytokine oncostatin M or a mutant form of human oncostatin M and the effector domain with cytotoxic activity, such as the Pseudomonas exotoxin A.

In this arrangement according to the invention, oncostatin M or a mutant form of oncostatin M is a carrier directing the toxin molecule on a neoplastic cell through recognising and binding the oncostatin M-specific receptors. On the other hand, the Pseudomonas toxin domain is a cytotoxic effector which kills the target cell after the internalisation of the entire construct in the process of receptor(s) endocytosis.

The subject of the invention is a fusion protein comprising:

- domain (a) which is a fragment of the amino acid sequence of oncostatin M or of a mutant form of oncostatin M selected from the group consisting of SEQ. No 6, SEQ. No 7, SEQ. No 8, SEQ. No 9, and SEQ. No 10, and
- domain (b) which is the sequence of the Pseudomonas exotoxin A of SEQ. No 11.

Preferably, the fusion protein comprises:

- domain (a) which is a fragment of the amino acid sequence of oncostatin M of SEQ. No 6, and
- domain (b) which is the sequence of the Pseudomonas exotoxin A of SEQ. No 11.

Also preferably, the fusion protein comprises:

- domain (a) which is a fragment of the amino acid sequence of a mutant form of oncostatin M selected from the group consisting of SEQ. No 7, SEQ. No 8, and SEQ. No 10, and
- domain (b) which is the sequence of the Pseudomonas exotoxin A of SEQ. No 11.

Similarly preferably, the fusion protein comprises:

- domain (a) which is a fragment of the amino acid sequence of a mutant form of oncostatin M of SEQ. No 9, and
- domain (b) which is the sequence of the Pseudomonas exotoxin A of SEQ. No 11.

The subject of the invention is also the amino acid sequence of the so far undescribed mutant form of oncostatin M, presented by SEQ. No 9. In SEQ. No. 9 two substitutions are introduced to the oncostatin M sequence, namely Q20A / K163A, resulting in lowered affinity to receptors gp130 and OSMR/LIFR.

In the fusion protein according to the invention, domains (a) and (b) may be linked by a flexible linker such as, for example, the linker with a sequence GGGGSASGG of SEQ. No 12, or any other steric linker known to the person skilled in the art, e.g. glycine-serine linker or glycine-serine-alanine linker or single amino acid residue. In addition, fusion proteins according to the invention may be provided with a motif KDEL of SEQ. No 13 directing to the reticulum, or any other motif directing to the reticulum known to the person skilled in the art. These two features may additionally improve properties of the fusion proteins according to the invention, but are not critical to solve the technical problems of the invention.

Sequence No 11 of the Pseudomonas exotoxin mutant form is a sequence including amino acids 276 - 633 with deletion 390-405, of the sequence presented in NCBI Reference Sequence: WP_058170169.1.

Oncostatin M present in the construct will bind at least one of the receptors it recognises (gp130, OSMP, LIFR), which are often overexpressed on neoplastic cells.

In one embodiment of the fusion protein according to the invention, the sequence of oncostatin M of the domain (a) is presented as SEQ. No 6.

SEQ. No 6 is a wild version of oncostatin M, and specifically a sequence including amino acids 26-221 of the sequence deposited under the GenBank No AAH11589.1.

Such a variant is preferable for cells sensitive to oncostatin. The use of oncostatin M of SEQ. No 6 as a carrier of effectors enhances the cytotoxic effect of the fusion protein due to the fact that oncostatin M exhibits additional cytostatic effect. Such a variant of the fusion protein exhibits a synergistic effect derived from the activity of wild oncostatin M and from the Pseudomonas exotoxin.

The overrepresentation of OSM receptors on neoplastic cells is of dual importance to the present invention. Where the cytotoxic effect observed on a cell line is too strong, which could result in undesirable effects, according to the invention, mutant forms of oncostatin M with lowered affinity for one of its receptors are used in the fusion protein.

In such embodiment, the amino acid sequence of mutant forms of oncostatin M of domain (a) with lowered affinity for one of the receptors thereof is presented as SEQ. No 7 or SEQ. No 8. SEQ. No 7 is the sequence of oncostatin M with mutation Q20A with lowered affinity for the gp130 receptor.

SEQ. No 8 is the sequence of oncostatin M with mutation K163A with lowered affinity for the OSMR/LIFR receptor (Deller Mc et al. Crystal structure and functional dissection of the cytostatic cytokine oncostatin M. Structure. 2000 Aug 15;8(8):863-74).

As regard neoplasms on which oncostatin M has no effect, or even has a stimulating effect, or where toxicity appears under *in vivo* conditions, the elimination of biological activity thereof through the use, according to the invention, of mutant forms of oncostatin M with restricted affinity for one of its receptors in fusion proteins, enhances the safety of therapy without significant reduction in effectiveness.

The fusion protein according to the invention, comprising sequences of a mutant form of oncostatin M with lowered affinity for both gp130 and OSMR/LIFR receptors as the domain (a), is the negative control.

In this embodiment, the sequence of a mutant form of oncostatin M with lowered affinity for both gp130 and OSMR/LIFR receptors is presented as SEQ. No 9.

In another embodiment of the fusion protein according to the invention, the sequence of a mutant form of oncostatin M of the domain (a) is presented as SEQ. No 10.

SEQ. No 10 is a deletion variant of oncostatin M, in which a loop (between helices B and C) present in the vicinity of the site of binding of the OSMR and LIFR receptors has been removed from the structure. In this variant which in literature is referred to as M2 (Chollangi S. et al., A unique loop structure in oncostatin M determines binding affinity toward oncostatin M receptor and leukemia inhibitory factor receptor. J Biol Chem. 2012 Sep 21;287(39):32848-59), the modification involves removing amino acids 93 - 103, and replacing them with a short three-glycine (GGG) linker. Such a variant exhibits stronger receptor binding, and more intensely induces the inhibition of cell proliferation.

In particular, the subject of the invention is fusion proteins: OSMPE1.0 of SEQ. No 1, OSMPE1.1 of SEQ. No 2, OSMPE1.2 of SEQ. No 3, OSMPE1.3 of SEQ. No 4, OSMPE2.0 of SEQ. No 5, which are described in detail, respectively, in Embodiments 1 to 5.

**Examples of embodiments:**

**Example 1. Fusion protein OSMPE1.0 of SEQ. No 1**

The fusion protein consists of a wild version of oncostatin M of SEQ. No 6 (OSM: GenBank: AAH11589.1 amino acids 26-221) with the active domain of Pseudomonas endotoxin 276 - 633 (del 390-405) of SEQ. No 11 with point mutations in order to reduce immunogenicity and the KDEL motif of SEQ. No 13 for the effective transport to cytoplasmic reticulum. Oncostatin and the Pseudomonas toxin domains were linked by the flexible linker GGGGSASGG of SEQ. No 12.

SEQ. No 1:

```
  1    AAIGSCSKEY RVLLGQLQKQ TDLMQDTSRL LDPYIRIQGL DVPKLREHCR

 51    ERPGAFPSEE TLRGLGRRGF LQTLNATLGC VLHRLADLEQ RLPKAQDLER

101    SGLNIEDLEK LQMARPNILG LRNNIYCMAQ LLDNSDTAEP TKAGRGASQP

151    PTPTPASDAF QRKLEGCRFL HGYHRFMHSV GRVFSKWGES PNRSRRGGGG

201    SASGGPEGGS LAALTAHQAC HLPLETFTRH RQPRGWEQLE QCGYPVQRLV

251    ALYLAARLSW NQVDQVIANA LASPGSGGDL GEAIRESPEQ ARLALTLAAA

301    ESERFVRQGT GNDEAGAANG PADSGDALLE RNYPTGAEFL GDGGDVSFST

351    RGTQNWTVER LLQAHRQLEE AGYVFVGYHG TFLEAAQSIV FGGVRARSQD

401    LDAIWAGFYI AGDPALAYGY AQDQEPDAAG RIRNGALLRV YVPRSSLPGF

451    YATSLTLAAP EAAGEVERLI GHPLPLRLDA ITGPEESGGR LETILGWPLA

501    ERTVVIPSAI PTDPRNVGGD LDPSSIPDSE QAISALPDYA SQPGKPPKDE

551    L
```

OSM sequence: amino acids 1-196
Pseudomonas toxin sequence: amino acids 206-547
flexible linker and KDEL motif sequences are underlined

**Example 2. Fusion protein OSMPE1.1 of SEQ. No 2**

The fusion protein consists of oncostatin M with mutation Q20A of SEQ. No 7 which has lowered affinity for the gp130 receptor with the active domain of Pseudomonas endotoxin 276

- 633 (del 390-405) of SEQ. No 11 with point mutations in order to reduce immunogenicity and the KDEL motif of SEQ. No 13 for the effective transport to cytoplasmic reticulum. Oncostatin and the Pseudomonas toxin domains were linked by the flexible linker GGGGSASGG of SEQ. No 12.

SEQ. No 2:

```
  1    AAIGSCSKEY RVLLGQLQKA TDLMQDTSRL LDPYIRIQGL DVPKLREHCR
 51    ERPGAFPSEE TLRGLGRRGF LQTLNATLGC VLHRLADLEQ RLPKAQDLER
101    SGLNIEDLEK LQMARPNILG LRNNIYCMAQ LLDNSDTAEP TKAGRGASQP
151    PTPTPASDAF QRKLEGCRFL HGYHRFMHSV GRVFSKWGES PNRSRRGGGG
201    SASGGPEGGS LAALTAHQAC HLPLETFTRH RQPRGWEQLE QCGYPVQRLV
251    ALYLAARLSW NQVDQVIANA LASPGSGGDL GEAIRESPEQ ARLALTLAAA
301    ESERFVRQGT GNDEAGAANG PADSGDALLE RNYPTGAEFL GDGGDVSFST
351    RGTQNWTVER LLQAHRQLEE AGYVFVGYHG TFLEAAQSIV FGGVRARSQD
401    LDAIWAGFYI AGDPALAYGY AQDQEPDAAG RIRNGALLRV YVPRSSLPGF
451    YATSLTLAAP EAAGEVERLI GHPLPLRLDA ITGPEESGGR LETILGWPLA
501    ERTVVIPSAI PTDPRNVGGD LDPSSIPDSE QAISALPDYA SQPGKPPKDE
551    L
```

OSM sequence: amino acids 1-196
Pseudomonas toxin sequence: amino acids 206-547
flexible linker and KDEL motif sequences are underlined
mutations in the OSM sequence are emboldened

**Example 3. Fusion protein OSMPE1.2 of SEQ. No 3**

The fusion protein consists of oncostatin M with mutation K163A of SEQ. No 8 which has lowered affinity for the OSMR/LIFR receptor with the active domain of Pseudomonas endotoxin 276 - 633 (del 390-405) of SEQ. No 11 with point mutations in order to reduce immunogenicity and the KDEL motif of SEQ. No 13 for the effective transport to cytoplasmic reticulum. Oncostatin and the Pseudomonas toxin domains were linked by the flexible linker GGGGSASGG of SEQ. No 12

SEQ. No 3

```
  1    AAIGSCSKEY RVLLGQLQKQ TDLMQDTSRL LDPYIRIQGL DVPKLREHCR
 51    ERPGAFPSEE TLRGLGRRGF LQTLNATLGC VLHRLADLEQ RLPKAQDLER
101    SGLNIEDLEK LQMARPNILG LRNNIYCMAQ LLDNSDTAEP TKAGRGASQP
151    PTPTPASDAF QRALEGCRFL HGYHRFMHSV GRVFSKWGES PNRSRRGGGG
201    SASGGPEGGS LAALTAHQAC HLPLETFTRH RQPRGWEQLE QCGYPVQRLV
251    ALYLAARLSW NQVDQVIANA LASPGSGGDL GEAIRESPEQ ARLALTLAAA
301    ESERFVRQGT GNDEAGAANG PADSGDALLE RNYPTGAEFL GDGGDVSFST
351    RGTQNWTVER LLQAHRQLEE AGYVFVGYHG TFLEAAQSIV FGGVRARSQD
401    LDAIWAGFYI AGDPALAYGY AQDQEPDAAG RIRNGALLRV YVPRSSLPGF
451    YATSLTLAAP EAAGEVERLI GHPLPLRLDA ITGPEESGGR LETILGWPLA
501    ERTVVIPSAI PTDPRNVGGD LDPSSIPDSE QAISALPDYA SQPGKPPKDE
551    L
```

OSM sequence: amino acids 1-196
Pseudomonas toxin sequence: amino acids 206-547
flexible linker and KDEL motif sequences are underlined
mutations in the OSM sequence are emboldened

**Example 4. Fusion protein OSMPE1.3 of SEQ. No 4**

The fusion protein consists of oncostatin M with mutations Q20A / K163A of SEQ. No 9 which has lowered affinity for the gp130 and OSMR/LIFR receptors with the active domain of Pseudomonas endotoxin 276 - 633 (del 390-405) of SEQ. No 11 with point mutations in order to reduce immunogenicity and the KDEL motif of SEQ. No 13 for the effective transport to cytoplasmic reticulum. Oncostatin and the Pseudomonas toxin domains were linked by the flexible linker GGGGSASGG of SEQ. No 12. The protein is a control comprising modifications which do not allow the targeting domain to effectively interact with any receptor.

SEQ. No 4:

```
  1    AAIGSCSKEY RVLLGQLQKA TDLMQDTSRL LDPYIRIQGL DVPKLREHCR

 51    ERPGAFPSEE TLRGLGRRGF LQTLNATLGC VLHRLADLEQ RLPKAQDLER

101    SGLNIEDLEK LQMARPNILG LRNNIYCMAQ LLDNSDTAEP TKAGRGASQP

151    PTPTPASDAF QRALEGCRFL HGYHRFMHSV GRVFSKWGES PNRSRRGGGG

201    SASGGPEGGS LAALTAHQAC HLPLETFTRH RQPRGWEQLE QCGYPVQRLV

251    ALYLAARLSW NQVDQVIANA LASPGSGGDL GEAIRESPEQ ARLALTLAAA

301    ESERFVRQGT GNDEAGAANG PADSGDALLE RNYPTGAEFL GDGGDVSFST

351    RGTQNWTVER LLQAHRQLEE AGYVFVGYHG TFLEAAQSIV FGGVRARSQD

401    LDAIWAGFYI AGDPALAYGY AQDQEPDAAG RIRNGALLRV YVPRSSLPGF

451    YATSLTLAAP EAAGEVERLI GHPLPLRLDA ITGPEESGGR LETILGWPLA

501    ERTVVIPSAI PTDPRNVGGD LDPSSIPDSE QAISALPDYA SQPGKPPKDE

551    L
```

OSM sequence: amino acids 1-196
Pseudomonas toxin sequence: amino acids 206-547
flexible linker and KDEL motif sequences are underlined
mutations in the OSM sequence are emboldened

## Example 5. Fusion protein OSMPE2.0 of SEQ. No 5

The example in which oncostatin M was used in the form of a variant referred to in literature as M2, which has deletion of amino acids 93 - 103 and, in their place, the insertion of a short three-glycine linker. The variant should more intensely bind with OSMR receptor, and induce the inhibition of proliferation. To this variant of OSM, the active domain of the Pseudomonas exotoxin 276 - 633 (del 390-405) was added, with point mutations in order to reduce immunogenicity and the KDEL motif of SEQ. No 13 for the effective transport to cytoplasmic reticulum, linked by the flexible linker GGGGSASGG of SEQ. No 12

The fusion protein consists of oncostatin M in the form of the so-called variant M2 of SEQ. No 10, which has deletion of amino acids 93 - 103 and, in their place, the insertion of a short three-glycine linker, which variant more intensely binds with OSMR receptor, and induces the inhibition of proliferation and of the active domain of the Pseudomonas endotoxin 276 - 633 (del 390-405) of SEQ. No 11 with point mutations in order to reduce immunogenicity, and the KDEL motif of SEQ. No 13 for the effective transport to cytoplasmic reticulum. Oncostatin and the Pseudomonas toxin domains are linked by the flexible linker GGGGSASGG of SEQ. No 12.

SEQ. No 5:

```
  1  AAIGSCSKEY RVLLGQLQKQ TDLMQDTSRL LDPYIRIQGL DVPKLREHCR

 51  ERPGAFPSEE TLRGLGRRGF LQTLNATLGC VLHRLADLEQ RLGGGNIEDL

101  EKLQMARPNI LGLRNNIYCM AQLLDNSDTA EPTKAGRGAS QPPTPTPASD

151  AFQRKLEGCR FLHGYHRFMH SVGRVFSKWG ESPNRSRRGG GGSASGGPEG

201  GSLAALTAHQ ACHLPLETFT RHRQPRGWEQ LEQCGYPVQR LVALYLAARL

251  SWNQVDQVIA NALASPGSGG DLGEAIRESP EQARLALTLA AAESERFVRQ

301  GTGNDEAGAA NGPADSGDAL LERNYPTGAE FLGDGGDVSF STRGTQNWTV

351  ERLLQAHRQL EEAGYVFVGY HGTFLEAAQS IVFGGVRARS QDLDAIWAGF

401  YIAGDPALAY GYAQDQEPDA AGRIRNGALL RVYVPRSSLP GFYATSLTLA

451  APEAAGEVER LIGHPLPLRL DAITGPEESG GRLETILGWP LAERTVVIPS

501  AIPTDPRNVG GDLDPSSIPD SEQAISALPD YASQPGKPPK DEL
```

OSM sequence: amino acids 1-188
Pseudomonas toxin sequence: amino acids 198-539
flexible linker and KDEL motif sequences are underlined
mutations in the OSM sequence are emboldened.

**Example 6. Preparation of fusion protein preparations**

The amino acid sequences of the fusion proteins described above in Examples from 1 to 5 (i.e. the sequences SEQ. No 1, SEQ. No 2, SEQ. No 3, SEQ. No 4, SEQ. No 5) served as matrices for generating the DNA sequences (which code them) containing codons optimized for the expression in E.coli when using reverse translation tools available on-line from the portal expassy when using default program settings in order to obtain optimum codons to be expressed in E.coli, with a preference for human codons.

The obtained nucleotide sequence were then automatically synthesised with the additionally attached to them sites for NdeI restriction enzymes (on the 5' end of the leading strand) and XhoI (on the 3' end of the leading strand). These sequences were used for the cloning of the gene of appropriate fusion protein to the vector derived from pET28a (Novagen). Proteins from such a construct had, on the N end, a polyhistidine tag (10 histidines), preceded by the polyglycine sequence and the site recognised by thrombin, which was subsequently used for the purification thereof using affinity chromatography. Correctness of the obtained constructs was confirmed by the automatic sequencing of the entire reading frame.

The obtained plasmids were used for the overproduction of proteins in the E.coli strain BL21 DE3 RIL Codon-Plus (Startagen). The above strain was transformed with them, and the colonies obtained on a selecting medium (LB agar, kanamycin, chloramphenicol, 1% glucose) were used for setting up an overnight culture in the liquid LB medium supplemented with kanamycin, chloramphenicol, and glucose. Pre-cultures were used to inoculate the appropriate culture. They were conducted in the TB medium supplemented with kanamycin and chloramphenicol at a temperature of 37ºC. At OD 0.15-0.5, the culture was induced with 1 mM IPTG, and the temperature was lowered to 25°C. The expression was carried out for 18 hours. The bacterial pellet was collected by centrifugation of the culture at 7000 g, and analysed electrophoretically using SDS-PAGE

The cells after the overproduction were desintegrated in a buffer with the following composition: 100 mM Tris-HCI, 300 mM NaCl, 10 mM Imidazol, pH 8. The obtained extract was clarified by centrifugation for 50 minutes at 8000 g. Supernatant was loaded onto His Ni-NTA column. In order to elute fractions containing proteins which do not bind to the resin, the column was rinsed with 3 column volumes of the buffer with the composition as provided above. In order to wash out the specifically bound proteins, the resin was rinsed with 10 volumes of 0-100% linear gradient buffer with the composition as provided above, additionally containing 500 mM imidazole. The collected fractions were analysed using SDS-PAGE.

The fractions exhibiting the presence of proteins with the expected weight were combined and digested with thrombin (in a ratio of 1 U of thrombin per 4 mg of protein for 8 hours at 16°C) in order to remove the polyhistidine tag, and then dialised to the formulation buffer: PBS (10 mM Na3PO4, 137 mM NaCl, 2.7 mM KCl) + 2 mM reduced glutathione.

## Example 7. Analysis of fusion protein preparations

Concentration, purity, and the secondary structure were determined for the obtained fusion protein preparations (CD, circular dichroism). Then, purified fusion protein preparations were tested for specificity of interaction with oncostatin M receptors using the surface plasmon resonance method (BIACORE T200). The gp130 and OSMR receptors (recombinant preparations obtained by standard methods) were immobilised on the CM5 sensor chip using amine bonds to the level of approx. 2000 RU (reflection units). The tested fusion protein preparations according to the invention and the reference ones were injected at increasing concentrations, and the bound fraction was dissociated in uniform time. The obtained signals were analysed using T200 BiaEvaluation software. For the calculations, a standard Langmuir 1:1 model was selected. This enabled the determination of association and dissociation constants Kon and Kof, and of the large dissociation constant determining the strength of the KD complex. The lower the KD value, the stronger the determined bond is.

In order to verify affinity of selected fusion proteins according to the invention for oncostatin M receptors, interaction kinetics parameters were measured for them. The results are presented in Table 2. The values of the constants for the wild form of oncostatin M (not subjected to coniugation) in relation to each OSM receptor, and for the fusion molecule OSMPE1.1 according to Example 2, which has been mutated in order to obtain lowered affinity for the gp130 receptor, were calculated. The obtained KD value for the gp130 receptor is greater than the value obtained for the wild OSM by over two orders of magnitude, which indicates the kinetics of interaction with this receptor being slower by over two orders of magnitude, as compared to the variant of wild oncostatin M. The determined kinetic values confirm that the fusion protein OSMPE1.1 according to Example 2 enables the lowering of affinity for the receptors for OSM. Therefore, it can be predicted that on the cell and animal model, totally lowered cytotoxic activity will be obtained (as compared to the use of the wild OSM as the toxin carrier). Therefore, the use of a mutant form of OSM with lowered affinity for the OSM receptors as a carrier enables the modulation of the cytotoxic response.

Table 2. Examples of results of testing kinetic parameters of interaction of the protein fusion OSMPE1.1 according to Example 2, and of the reference wild OSM preparation, with the gp130 and OSMR receptors. The tests were carried out using the surface plasmon resonance method on a BIACORE T200.

| Fusion protein/receptor | Ka (1/Ms) | Kd (1/s) | KD (M) |
|---|---|---|---|
| OSM/gp130 | 2.13E+04 | 1.95E-03 | 9.13E-08 |
| OSM/OSMR | 6.27E+04 | 3.92E-03 | 6.25E-07 |
| OSMPE1.1/gp130 | 3.75E+3 | 9.88E-03 | 2.63E-5 |
| OSMPE1.1/OSMR | 8.39E+04 | 2.07E-03 | 2.46E-08 |

Designations:

OSM - reference of the wild oncostatin M itself (recombinant protein),
OSMPE1.1 - fusion protein according to Example 2 (with lowered affinity for the gp130 receptor)

## Example 9. Cytotoxicity analysis

Next, the fusion proteins according to the invention from Examples from 1 to 5 served for the cytotoxicity testing on a panel of 10 human cell lines (9 neoplastic lines: A549, SK-MES-1, A431, A375, SKOV3, HCT116, HepG2, Panc1, MiaPaCa2 from various organs, and one non-neoplastically transferred line of human fibroblasts CCD11Lu, serving as the reference of activity towards normal cells), and for the determination of the potential risk of general toxicity.

The cell lines were acquired from the ATCC, and then propagated and deposited at the ADAMED's Biological Laboratory Cell Line Bank. The cultures were maintained under standard conditions: 37°C, 5% CO2, 95% humidity, and were cultured in mediums optimum for them in accordance with the ATCC recommendations for particular lines. The cell culture was diluted with the culture medium to the specified density (approx. $3 \times 10^5$ - cells per 1 ml). Then, 150 $\mu$l of appropriately diluted cell suspension were loaded on a 96-well plate in thriplicates. The thus prepared cells were incubated for 24 hours at 37°C in 5% CO2, 95% humidity, wherein to the cells suspended in 150 $\mu$l of culture medium

suitable for them, another 50 µl of appropriate culture medium was added, which contained various concentrations of appropriate tested fusion protein according to the invention, obtained according to the procedure described above, or control. The cells were incubated with protein preparations for the next 72 hours, and then, 20 µl of standard, commercially available MTT working solution were added to the culture medium with the test preparations (5 mg/ml), and incubated for 3 hours at 37°C in 5% CO2. Then, the culture medium with the MTT solution was removed, and the formazan crystals were dissolved by adding 100 µl DMSO. After stirring, absorbance was measured at the wavelength of 570 nm (reference filter 690 nm), and the results were translated into the vitality of the culture in relation to the untreated control, and on this basis, the IC50 value was determined (the concentration inhibiting the vitality of cells in 50%).

Cytotoxicity testing results are summarised in Table 1.

Fusion proteins designed as described in Examples 1 to 5 and prepared as described in Example 6, but with the difference that different linkers (GGGGS, GGGS, GGG,GGGG, GGSGG, GGSG, GSG, SGG, ASGG, ASG, GGGSASGG, GGSGGGSGGG, GGSGGGGGS, GGGGGGS, SHHS and single glycine and alanine residues) and reticulum directing domein (KEDL), were also subjected to cytotoxicity tests on cell lines . However, the minor changes in cytotoxicity of these fusions agains neoplastic cell lines were not statistically important.

Table 1. Results of cytotoxicity testing on cell lines (averaged IC50 results from three independent measurements).

| Protein | Lungs | | Skin | | Ovary | Colon | Liver | Pancreas | | Fibrobla sts |
|---|---|---|---|---|---|---|---|---|---|---|
| | A549 | SK-MES-1 | A431 | A375 | SKOV3 | HCT116 | HepG2 | Panc1 | Mia PaCa2 | CCD11L u |
| OSMPE1.0 Ex. 1 | 30,09 | 21,14 | 531,53 | >9000 | 2678,00 | 2476,00 | 16,30 | 7,75 | 4,41 | 7345,50 |
| OSMPE1.1 Ex. 2 | 127,13 | 114,88 | 863,50 | 9259,67 | 1767,33 | 1569,00 | 31,37 | 39,18 | 60,39 | 9627,33 |
| OSMPE1.2 Ex. 3 | 211,00 | 222,87 | 2214,00 | >17500 | 2963,00 | 2154,00 | 90,30 | 224,43 | 100,81 | 6467,00 |
| OSMPE1.3 Ex. 4 | 573,57 | 1392,00 | 2042,00 | >12500 | 8197,00 | 3367,00 | 247,20 | 253,27 | 278,27 | >12500 |
| OSMPE2.0 Ex. 5 | 10,52 | 6,51 | 305,77 | 1432,50 | 313,93 | 313,83 | 2,02 | 5,58 | 2,92 | 4446,67 |
| Wild OSM | 1744,33 | 3188,00 | 2211,00 | >17500 | 10397,3 3 | 8641,00 | 16222,50 | 4222,67 | 759,67 | >17500 |
| PE2.0 | >20000 | >20000 | >20000 | 6136,50 | >20000 | >20000 | >20000 | 592,97 | >2000 0 | 545,10 |

Designations in the table:

In line 1, the origin of neoplastic cell lines is provided, and in line 2, their code designations according to the ATCC are shown.

The Human Fibroblast Line is the reference of non-neoplastically transformed (normal) cells. OSM - reference: wild oncostatin M,

PE2.0 - reference: the Pseudomonas exotoxin A domain itself, identical to the one used in fusion proteins according to the invention, (SEQ. No 11)

OSMPE1.0.- according to Example 1 (wild version of OSM with the PE toxin), OSMPE1.1 - according to Example 2 (lowered affinity for the gp130 receptor), OSMPE1.2 - according to Example 3 (lowered affinity for the OSMR/LIFR receptor), OSMPE1.3 - according to Example 4 (lowered affinity for both receptors - negative control), OSMPE2.0 - according to Example 5 (deletion in OSM 93-103, increased affinity for the OSMR receptor).

IC50 values are given in ng/ml. It is important to compare the change in the obtained IC50 values in relation, separately, to each cell line used, which differ in the level of expression of the receptors targeted at by the fusion proteins according to the invention.

Tests on cell lines using the recombinant protein of wild oncostatin M as well as the Pseudomonas exotoxin A, used individually, demonstrated that they exhibited unsatisfactory toxicity (high IC50 values), and no inhibition of the neoplastic tumour growth was observed on an animal model, and moreover, as regards the exotoxin, high systemic toxicity.

The results summarised in Table 1 clearly show that the fusion protein components used separately do not produce cytotoxic effect (measured by the IC50 value), since high IC50 values were calculated by only extrapolating the curve for lower, acceptable concentration levels for experiments of this type.

The fusion molecules according to the invention exhibit, in most cases (depending on the mutant form of oncostatic M), IC50 values significantly lower than their components, therefore the fusion proteins according to the invention have generated a synergistic effect.

Fusion of the wild variant of OSM according to Example 1 exhibits higher activities (lower IC50) than mutant forms with lowered affinities for specific receptors (fusions according to Examples 2, 3 and 4), wherein the variant with the double-lowered affinity for both the gp130 and OSMR (OSMPE1.3-Example 4) has the weakest affinity.

The variant OSMPE2.0 - of Example 5, which, in the OSM gene, has deletion which increases affinity for the OSMR receptor, and exhibits the highest activities (the lowest IC50).

All tested fusion proteins according to the invention exhibit high IC50 values in relation to human fibroblasts (from several to several hundred times higher than those on neoplastic cells), which confirms low specificity and, consequently, toxicity of the molecules towards normal cells.

It is possible to indicate neoplastic cell lines particularly sensitive to fusion proteins according to the invention, derived from the lungs and pancreas, which indirectly correlates with the cases of sensitivity to oncostatin, described in literature. This sensitivity most probably correlates with the level of expressed receptors for OSM; however, there are no detailed data specifying the levels of expression of OSM receptors for particular types of neoplasms.

The use of fusion molecules of oncostatin M or mutant forms thereof with restricted affinity according to the invention, but still binding at least one OSM receptor with a non-specific cytotoxic domain of the Pseudomonas exotoxin, enables the modulation of the toxin activity level, and thus reduces the risk of additional toxic effects in the case of neoplasms exhibiting particular sensitivity to OSM. The use of fusion proteins according to the invention significantly increases antineoplastic efficacy as compared to the component fusion proteins used separately. Tests on a cell panel indicate the emergence of examples of a specific cytotoxic effect exerted on neoplastic cells by fusion molecules as compared to oncostatin M itself, in the absence of activity on the non-transformed cells.

In the case of lowered affinity of fusion proteins according to the invention for specific types of neoplasms or neoplastic lines on which OSM may induce proliferation, the use of a strong effector such as the Pseudomonas toxin is sufficient to trigger selective toxicity over the tumour growth signal on these cells. Where the action of cytostatic effector is, for a particular type of neoplasm, unsatisfactory for inhibiting the pro-oncogenic activity of OSM, the use of variants with lowered affinity for one of the receptors (e.g. gp130) blocks this activity (inhibition of the formation of a biologically active complex), and enables the action of the toxin effector itself, which is targeted at the neoplasm. In addition, such a form of the protein will be an antagonist of the physiologically secreted oncostatin M.

The use of fusion proteins according to the invention with lowered affinity for the OSM receptors (OSMPE2.0 - according to Example 5) ensures the obtaining of cytotoxic effect in the case of cells which will not respond to OSM, or were characterised by a lowered level of the presented receptors for OSM.

Fusion proteins according to the invention, comprising oncostatin M and mutant forms thereof with modified affinity for receptors and the non-specific domain of the cytotoxic Pseudomonas exotoxin, reduce the risk of systemic effects, and significantly enhance the effectiveness of the toxin molecule itself. Tests on a cell panel indicate the emergence of examples of a specific cytotoxic effect exerted on neoplastic cells by fusion proteins according to the invention as compared to oncostatin M itself and to the Pseudomonas toxin itself, in the absence of activity on the non-transformed cells.

**Example 10. Antineoplastic efficacy of fusion proteins *in vivo* on xenografts**

Antineoplastic activity of protein preparations was tested on a murine model of human lung neoplasm A549.

Cells of human lung neoplasm A549 were maintained in culture medium RPMI1640 (HyClone, Logan, UT, USA) supplemented with 10% foetal calf serum and 2 mM glutamine. On the day of mice grafting, the cells were unstuck from the medium by washing with trypsin (Invitrogen), then centrifuged at 1300 rpm, 4°C, 8 minutes, and then suspended in HBSS buffer (Hanks medium).

Testing for antineoplastic activity of proteins was conducted on 4-5 week old mouse males of the Crl:SHO-Prkdc$^{scid}$Hr$^{hr}$ strain, acquired from the Charles River Germany mouse breeding facility. The mice were kept under conditions free from specific pathogens, with feed and demineralised water provided *ad libitum.* All experiments on animals were conducted in accordance with the guidelines "Interdisciplinary Principles and Guidelines for the Use of Animals in Research, Marketing and Education" issued by the New York Academy of Sciences' Ad Hoc Committee on Animal Research, and were accepted by the 4th Local Ethical Committee for Experiments on Animals in Warszawa.

The size of the tumour was measured with an electronic caliper, and the volume of the tumour was calculated according to the following formula: $(a^2 \times b)/2$, where a = the shorter diagonal of the tumour (mm), and b = the longer diagonal of the tumour (mm). Tumour growth inhibition was calculated using the following formula:

$$\text{TGI (\%)} \, (\textit{tumour growth inhibition}) = (\text{WT/WC}) \times 100 - 100\%,$$

where WT denotes the average volume of the tumour in the treated group, and WC denotes the average volume of the tumour in the control group.

Results of the experiments are presented as an average value +/- SD (SD). All calculations and graphs were prepared using GraphPad Prism 5.0 software.

Lung neoplasm model: On day 0, the mice were grafted using a syringe with a 0.5 x 25 mm needle (Bogmark) subcutaneously (s.c.) on the right side of the body with cells at an amount of 5x106 cells of human lung neoplasm A549 suspended in 0.1 ml mixture of HBSS buffer and Matrigel (4:1). In day 20 of the experiment, the mice were randomised so that the average size of tumours in a group was 175 mm$^3$, and three animals were assigned to each group. In the groups, fusion protein preparations according to the invention from Example 5 (0.1 mg/kg) and, comparatively, human recombinant Oncostatin M (0.1 mg/kg) and the recombinant active domain of *Pseudomonas aeruginosa* exotoxin A, as well as physiological saline solution as the control, were administered. The preparations were administered intravenously (*i.v.*) according to the following regimen: everyday administration for 10 days. On day 32 of the experiment, the mice were sacrificed by spinal cord disruption.

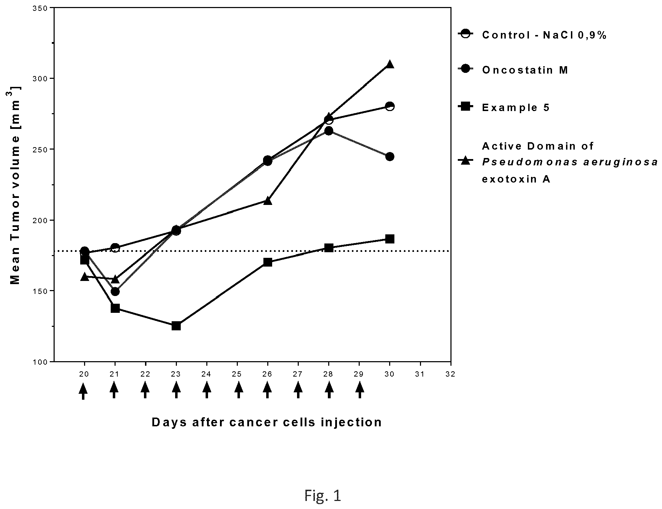

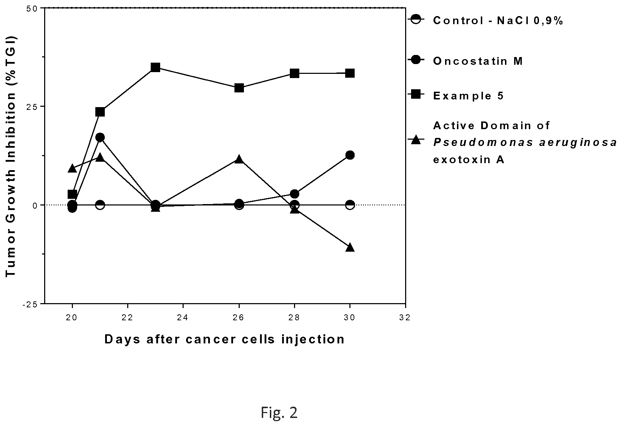

The experiment results are presented in Fig. 1 and Fig. 2, which show changes in the neoplastic tumour volume in relation to the control (Fig. 1), and the tumour growth inhibition (% TGI) as a percentage of the control (Fig. 2) in mice.

As shown by the experiment results, the administration of the fusion protein according to the invention from Example 5 resulted in the inhibition of the growth of human lung neoplasm A549 tumour, with the TGI value of 39.5% in relation to the control on day 23, maintained at a similar level for the rest of the experiment. For Oncostatin M and the *Pseudomonas aeruginosa* exotoxin A active domain, used as reference preparations, maximum point TGI values at a level of, respectively, 15.9% and 11.7% were obtained. Therefore, the fusion protein according to the invention exhibits stronger action as compared to its components using separately, and, therefore, a synergy effect was obtained for the fusion protein according to the invention from Example 5.

Due to the differentiation of cell targets for oncostatin, OSM may be used in a targeted cell therapy as a carrier with pleiotropic activity of binding with neoplastic cells. In addition, the involvement of OSM in various cell mechanisms results in the obtaining of synergistic effects. These effects are expressed by the obtaining, for the fusion proteins according to the invention, a cytotoxic effect towards the cells of neoplastic lines, and by the obtaining of % inhibition of tumour growth on an animal model being higher than the total effects obtained as a result of the use of single components, and also being higher than in the case of the use of a mixture of these components.

**Claims**

1. A fusion protein comprising:

   - domain (a) which is a fragment of the amino acid sequence of oncostatin M or of a mutant form of oncostatin M selected from the group consisting of SEQ. No 6, SEQ. No 7, SEQ. No 8, SEQ. No 9, and SEQ. No 10, and
   - domain (b) which is the sequence of the Pseudomonas exotoxin A of SEQ. No 11.

**2.** The fusion protein according to Claim 1, wherein domain (a) is SEQ. No 6.

**3.** The fusion protein according to Claim 1, wherein domain (a) is selected from among SEQ. No 7, SEQ. No 8, and SEQ. No 9.

**4.** The fusion protein according to Claim 3, wherein domain (a) is selected from among SEQ. No 7 and SEQ. No 8.

**5.** The fusion protein according to Claim 3, wherein domain (a) is SEQ. No 9.

**6.** The fusion protein according to Claim 1, wherein domain (a) is SEQ. No 10.

**7.** Fusion protein according to Claim 1 with the amino acid sequence selected from the group consisting of SEQ. No 1, SEQ. No 2, SEQ. No 3, SEQ. No 4 and SEQ. No 5.

**8.** A mutant form of oncostatin M with the amino acid sequence SEQ. No 9


**Patentansprüche**

**1.** Fusionsprotein, enthaltend:

- Domäne (a), bei der es sich um ein Fragment der Aminosäuresequenz von Oncostatin M oder der mutierten Form von Oncostatin M handelt, ausgewählt aus der Gruppe bestehend aus der Sequenz (SEQ) Nr.6, Sequenz Nr. 7, Sequenz Nr. 8, Sequenz Nr. 9 und Sequenz Nr. 10; und
- Domäne (b), bei der es sich um die Sequenz von Exotoxine A aus dem Bakterium Pseudomonas aeruginosa der Sequenz Nr. 11 handelt

**2.** Fusionsprotein nach Anspruch 1, **dadurch gekennzeichnet, dass** es sich bei der Domäne (a) um die Sequenz Nr. 6 handelt

**3.** Fusionsprotein nach Anspruch 1, **dadurch gekennzeichnet, dass** die Domäne (a) aus der Sequenz Nr. 7, der Sequenz Nr. 8 und der Sequenz Nr. 9 ausgewählt wird

**4.** Fusionsprotein nach Anspruch 3, **dadurch gekennzeichnet, dass** die Domäne (a) aus der Sequenz Nr. 7 und der Sequenz Nr. 8 ausgewählt wird

**5.** Fusionsprotein nach Anspruch 3, **dadurch gekennzeichnet, dass** es sich bei der Domäne (a) um die Sequenz Nr. 9 handelt

**6.** Fusionsprotein nach Anspruch 1, **dadurch gekennzeichnet, dass** es sich bei der Domäne (a) um die Sequenz Nr. 10 handelt

**7.** Fusionsprotein nach Anspruch 1, **dadurch gekennzeichnet, dass** die Aminosäuresequenz aus einer Gruppe bestehend aus der Sequenz Nr. 1, Sequenz Nr. 2, Sequenz Nr. 3, Sequenz Nr. 4 und Sequenz Nr. 5 ausgewählt wird

**8.** Die mutierte Form von Oncostatin M mit der Aminosäuresequenz SEQ Nr. 9


**Revendications**

**1.** Protéine de fusion contenant :

- un domaine (a) qui est un fragment d'une séquence d'acides aminés de l'oncostatine M ou d'une forme mutante de l'oncostatine M choisie dans le groupe constitué par la séquence (SEQ) n° 6, la séquence n° 7, la séquence n° 8, la séquence n° 9 et la séquence n° 10 ; et
- un domaine (b) qui est la séquence de l'exotoxine A de Pseudomonas aeruginosa (bacille du pus bleu) séquence n° 11

**2.** Protéine de fusion selon la revendication 1, **caractérisée en ce que** le domaine (a) est la séquence n° 6

**3.** Protéine de fusion selon la revendication 1, **caractérisée en ce que** le domaine (a) est choisi parmi la séquence n° 7, la séquence n° 8 et la séquence n° 9

**4.** Protéine de fusion selon la revendication 3, **caractérisée en ce que** le domaine (a) est la séquence n° 7

**5.** Protéine de fusion selon la revendication 3, **caractérisée en ce que** le domaine (a) est la séquence n° 9

**6.** Protéine de fusion selon la revendication 1, **caractérisée en ce que** le domaine (a) est la séquence n° 10

**7.** Protéine de fusion selon la revendication 1, **caractérisée en ce que** la séquence d'acides aminés est choisie dans le groupe constitué par la séquence n° 1, la séquence n° 2, la séquence n° 3, la séquence n° 4 et la séquence n° 5

**8.** Forme mutante de l'oncostatine M avec la séquence d'acides aminés SEQ n° 9

Fig. 1

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **W. DEBINSKI et al.** *J Biol Chem.,* 14 July 1995, vol. 270 (28), 16775-80
- **LIU-CHITTENDEN Y.** *Cancer Med.,* July 2015, vol. 4 (7), 1060-8
- **BOAYUE KB et al.** *Leukemia,* February 1998, vol. 12 (2), 182-91
- **ZARLING JM et al.** Oncostatin M: a growth regulator produced by differentiated histiocytic lymphoma cells. *Proc Natl Acad Sci U S A.,* December 1986, vol. 83 (24), 9739-43
- **DAVID E et al.** Oncostatin M is a growth factor for Ewing sarcoma. *Am J Pathol.,* November 2012, vol. 181 (5), 1782-95
- **TAMURA S. et al.** Developmental expression pattern of oncostatin M receptor beta in mice. *Mech Dev.,* July 2002, vol. 115 (1-2), 127-31
- **TANAKA M et al.** A multifunctional cytokine. *Rev Physiol Biochem Pharmacol.,* 2003, vol. 149, 39-52
- **CAFFAREL MM et al.** Oncostatin M receptor is a novel therapeutic target in cervical squamous cell carcinoma. *J Pathol.,* March 2014, vol. 232 (4), 386-90
- **LIU J. et al.** Interactions between oncostatin M and the IL-6 signal transducer, gp130. *Cytokine,* May 1994, vol. 6 (3), 272-8
- **HORN D. et al.** Regulation of cell growth by recombinant oncostatin M. *Growth Factors,* 1990, vol. 2 (2-3), 157-65

- **LI C et al.** Induction of S100A9 gene expression by cytokine oncostatin M in breast cancer cells through the STAT3 signaling cascade. *Breast Cancer Res Treat.,* September 2004, vol. 87 (2), 123-34
- **DAVID E et al.** Direct anticancer effect of oncostatin M on chondrosarcoma. *Int J Cancer.,* 15 April 2011, vol. 128 (8), 1822-35
- **GODOY-TUNDIDOR S. et al.** Interleukin-6 and oncostatin M stimulation of proliferation of prostate cancer 22Rv1 cells through the signaling pathways of p38 mitogen-activated protein kinase and phosphatidylinositol 3-kinase. *Prostate,* 01 July 2005, vol. 64 (2), 209-16
- **LI Q et al.** Oncostatin M promotes proliferation of ovarian cancer cells through signal transducer and activator of transcription 3. *Int J Mol Med.,* July 2011, vol. 28 (1), 101-8
- **DELLER MC et al.** Crystal structure and functional dissection of the cytostatic cytokine oncostatin M. *Structure,* 15 August 2000, vol. 8 (8), 863-74
- **CHOLLANGI S. et al.** A unique loop structure in oncostatin M determines binding affinity toward oncostatin M receptor and leukemia inhibitory factor receptor. *J Biol Chem.,* 21 September 2012, vol. 287 (39), 32848-59
- Interdisciplinary Principles and Guidelines for the Use of Animals in Research, Marketing and Education. New York Academy of Sciences